(19) Europäisches Patentamt
European Patent Office
Office européen des brevets

(11) **EP 3 640 743 B1**

(12) **FASCICULE DE BREVET EUROPEEN**

(45) Date de publication et mention de la délivrance du brevet:
**03.11.2021 Bulletin 2021/44**

(51) Int Cl.:
**G03H 1/08** *(2006.01)*  **G03H 1/04** *(2006.01)*
**G03H 1/00** *(2006.01)*  **G01N 15/14** *(2006.01)*

(21) Numéro de dépôt: **19203484.1**

(22) Date de dépôt: **16.10.2019**

(54) **PROCÉDÉ D'OBSERVATION D'UN ÉCHANTILLON**

BEOBACHTUNGSVERFAHREN EINER PROBE

METHOD FOR OBSERVING A SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.10.2018 FR 1859618**

(43) Date de publication de la demande:
**22.04.2020 Bulletin 2020/17**

(73) Titulaire: **Commissariat à l'Énergie Atomique et aux Énergies Alternatives**
**75015 Paris (FR)**

(72) Inventeurs:
• **HERVE, Lionel**
**38700 Corenc (FR)**
• **ALLIER, Cédric**
**38000 Grenoble (FR)**

(74) Mandataire: **Le Goaller, Christophe**
**Innovation Competence Group**
**310 avenue Berthelot**
**69372 Lyon Cedex 08 (FR)**

(56) Documents cités:
**US-A1- 2017 059 468    US-A1- 2017 212 343**

• OLIVIER THOMAS ET AL: "Optimizing phase object reconstruction using an in-line digital holographic microscope and a reconstruction based on a Lorenz-Mie model", PROCEEDINGS OF SPIE; [PROCEEDINGS OF SPIE ISSN 0277-786X VOLUME 10524], SPIE, US, vol. 10677, 24 mai 2018 (2018-05-24), pages 106772J-106772J, XP060109011, DOI: 10.1117/12.2311321 ISBN: 978-1-5106-1533-5
• KRISHNATREYA B J ET AL: "Measuring Boltzmann's constant through holographic video microscopy of a single colloidal sphere", AMERICAN JOURNAL OF PHYSICS, AMERICAN ASSOCIATION OF PHYSICS TEACHERS, US, vol. 82, no. 1, 31 décembre 2013 (2013-12-31), pages 23-31, XP009512632, ISSN: 0002-9505, DOI: DOI:10.1119/1.4827275

EP 3 640 743 B1

**Description**

## DOMAINE TECHNIQUE

Le domaine technique de l'invention est lié à l'observation d'un échantillon, en particulier un échantillon biologique, par imagerie sans lentille, en mettant en œuvre un algorithme de reconstruction holographique aux performances améliorées.

## ART ANTERIEUR

L'observation d'échantillons, et en particulier des échantillons biologiques, par imagerie sans lentille connaît un développement important depuis ces dix dernières années. Cette technique permet d'observer un échantillon en le disposant entre une source de lumière et un capteur d'image, sans disposer de lentille de grossissement optique entre l'échantillon et le capteur d'image. Ainsi, le capteur d'image collecte une image de l'onde lumineuse transmise par l'échantillon.

Cette image est formée de figures d'interférence entre l'onde lumineuse émise par la source de lumière et transmise par l'échantillon, et des ondes de diffraction, résultant de la diffraction par l'échantillon de l'onde lumineuse émise par la source de lumière. Ces figures d'interférences sont parfois dénommées figures de diffraction, ou désignées par le terme anglais « diffraction pattern ».

Le document WO2008090330 décrit un dispositif permettant l'observation d'échantillons biologiques, en l'occurrence des cellules, par imagerie sans lentille. Le dispositif permet d'associer, à chaque cellule, une figure d'interférence dont la morphologie permet d'identifier le type de cellule. L'imagerie sans lentille apparaît alors comme une alternative simple, et peu onéreuse, à un microscope classique. De plus, son champ d'observation est nettement plus important que ne peut l'être celui d'un microscope. On comprend alors que les perspectives d'application liées à cette technologie sont importantes.

D'une façon générale, l'image formée sur le capteur d'image, comportant des figures d'interférence, peut être traitée par un algorithme de reconstruction holographique, de manière à estimer des propriétés optiques de l'échantillon, par exemple un facteur de transmission ou une phase. De tels algorithmes sont bien connus dans le domaine de la reconstruction holographique. Pour cela, la distance entre l'échantillon et le capteur d'image étant connue, on applique un algorithme de propagation, prenant en compte cette distance, ainsi que la longueur d'onde de l'onde lumineuse émise par la source de lumière. On peut alors reconstituer une image d'une propriété optique de l'échantillon. L'image reconstruite peut, en particulier, être une image complexe de l'onde lumineuse transmise par l'échantillon, comportant des informations sur les propriétés optiques d'absorption ou de variation de phase de l'échantillon. Mais les algorithmes de reconstruction holographiques peuvent induire un bruit de reconstruction dans l'image reconstruite, désigné par le terme de « twin image ». Cela est essentiellement dû au fait que l'image formée sur le capteur d'image ne comporte pas d'information relative à la phase de l'onde lumineuse atteignant ce capteur. De ce fait, la reconstruction holographique s'effectue sur la base d'une information optique partielle, basée uniquement sur l'intensité de l'onde lumineuse collectée sur le capteur d'image.

L'amélioration de la qualité de la reconstruction holographique fait l'objet de nombreux développements, en mettant en oeuvre des algorithmes fréquemment dénommés « Phase retrieval », permettant une estimation de la phase de l'onde lumineuse auquel le capteur d'image est exposé.

Un algorithme de reconstruction numérique est par exemple décrit dans US2012/0218379. Des algorithmes de reconstruction ont également été décrits dans WO2016189257 ou dans WO2017162985.

Le document US2017/059468 ainsi que la publication Krishnatreya B et al "Measuring Boltzmann's constant holographic video microscopy of a single colloidal sphere" adressent la caractérisation de particules à partir d'un hologramme. L'hologramme est confronté à des modélisations, basées sur un modèle de type Lorenz-Mie, pour obtenir des hologrammes modélisés. Le document US2017/059468 utilise des profils radiaux, établis à partir d'hologrammes modélisés, en tant que données d'entrée d'un algorithme d'intelligence artificielle supervisé. L'algorithme est ensuite utilisé pour caractériser des particules à partir de leurs hologrammes.

Le document Thomas O. "Optimizing phase object reconstruction using an in-line digital holographic microscope and a reconstruction based on a Lorenz-Mie model", SPIE, US, vol. 10677, 24 may 2018, décrit un procédé pour analyser des particules, selon lequel on compare un hologramme, formé dans le plan de l'échantillon, acquis selon une configuration défocalisée, avec une figure de diffraction modélisée par un modèle théorique, en l'occurrence le modèle de Lorentz-Mie.

Les inventeurs proposent un procédé d'observation d'un échantillon par une méthode d'imagerie holographique, le procédé comprenant une étape de reconstruction d'une image complexe de l'échantillon, sur la base de laquelle on peut obtenir une représentation spatiale de paramètres de l'échantillon.

**EXPOSE DE L'INVENTION**

Un premier objet de l'invention est un procédé d'observation d'un échantillon, l'échantillon s'étendant selon un plan de l'échantillon définissant des coordonnées radiales, le procédé comportant les étapes suivantes :

a) illumination de l'échantillon à l'aide d'une source de lumière, apte à émettre une onde lumineuse incidente se propageant vers l'échantillon selon un axe de propagation;

b) acquisition, à l'aide d'un capteur d'image, d'une image de l'échantillon, formée dans un plan de détection, l'échantillon étant disposé entre la source de lumière et le capteur d'image, de telle sorte que l'onde lumineuse incidente subit une différence de trajet optique, parallèlement à l'axe de propagation, en traversant l'échantillon ;

c) traitement de l'image acquise par le capteur d'image ;

le procédé étant caractérisé en ce que le traitement de l'image acquise comporte les étapes itératives suivantes:

i) prise en compte d'un ensemble de vecteurs de paramètres, décrivant l'échantillon, et respectivement définis en plusieurs coordonnées radiales, dans le plan de l'échantillon, chaque vecteur de paramètres étant associé à une coordonnée radiale, et comportant un terme représentatif d'un paramètre optique l'échantillon, au moins un paramètre optique étant une différence de trajet optique induite par l'échantillon à chaque coordonnée radiale;

ii) à partir des vecteurs de paramètres, formation d'une image complexe de l'échantillon dans le plan de l'échantillon ;

iii) application d'un opérateur de propagation à l'image complexe de l'échantillon formée dans le plan de l'échantillon, pour calculer une image de l'échantillon dans le plan de détection ;

iv) comparaison de l'image acquise lors de l'étape b) et de l'image calculée lors de l'étape iii), pour calculer un indicateur de validité;

v) mise à jour de l'ensemble des vecteurs de paramètres, de façon à faire tendre l'indicateur de validité vers une valeur prédéterminée ;

vi) réitération des étapes ii) à v) en prenant en compte les vecteurs de paramètres estimés lors de l'étape v).

L'étape v) peut comporter une détermination d'un gradient de l'indicateur de validité en fonction d'au moins un paramètre, de telle sorte que les vecteurs de paramètres sont mis à jour pour réduire l'indicateur de validité de l'itération suivante. L'étape v) peut mettre en œuvre un algorithme de type descente de gradient.

Chaque vecteur de paramètre peut comporter:

- au moins un terme représentatif d'une différence de trajet optique selon l'axe de propagation ;
- un terme représentatif de l'absorbance.

Selon un mode de réalisation, l'étape v) comporte un calcul d'un critère morphologique à partir des vecteurs de paramètres, le critère morphologique quantifiant un écart des vecteurs de paramètres, décrivant l'échantillon, par rapport à une contrainte morphologique de l'échantillon, de telle sorte que l'indicateur de validité est obtenu à partir d'une combinaison :

- d'un critère d'erreur, déterminé à partir d'une comparaison entre l'image acquise lors de l'étape b) et de l'image obtenue lors de l'étape iii) ;
- et du critère morphologique.

L'indicateur de validité peut être une somme, éventuellement pondérée, du critère d'erreur et du critère morphologique. Selon un mode de réalisation, l'indicateur de validité est une somme pondérée du critère d'erreur et du critère morphologique, la pondération variant entre au moins deux itérations successives.

Selon un mode de réalisation :

- lors de l'étape b), une image de l'échantillon est acquise dans différentes bandes spectrales ;
- au moins un vecteur de paramètres, ou chaque vecteur de paramètres, comporte au moins un paramètre optique défini dans une des bandes spectrales ;

le procédé étant tel que :

- lors de l'étape iii), l'image complexe de l'échantillon est propagée dans chaque bande spectrale, de façon à calculer, dans chaque bande spectrale, une image de l'échantillon;
- l'étape iv) comporte une comparaison, dans chaque bande spectrale, de l'image calculée lors de l'étape iii) et de l'image acquise lors de l'étape b) ;

- de telle sorte que l'indicateur de validité calculé dans l'étape iv) est calculé à partir des comparaisons effectuées lors de l'étape iv).

Selon un mode de réalisation :

- lors de premières itérations, un paramètres optique est considéré comme ne variant pas en fonction de la bande spectrale ;
- au-delà de premières itérations, le paramètre optique est considéré comme variable en fonction de la bande spectrale.

Lors de l'étape iii), le calcul de l'image dans le plan de détection peut notamment comporter une application d'un produit de convolution par un noyau de convolution, le noyau de convolution représentant une étendue spatiale de la source de lumière.

Lors de l'étape iii), le calcul de l'image dans le plan de détection peut comporter une intégrale d'une image élémentaire, définie dans une bande spectrale élémentaire, l'intégrale étant calculée pour tenir compte d'une bande spectrale d'émission de la source de lumière. La bande spectrale d'émission est alors formée par une juxtaposition de différentes bandes spectrales élémentaires. Autrement dit, chaque bande spectrale élémentaire forme la bande spectrale.

Un deuxième objet de l'invention est un dispositif d'observation d'un échantillon comportant :

- une source de lumière, configurée pour émettre une onde lumineuse incidente pour illuminer l'échantillon ;
- un support d'échantillon, configuré pour recevoir l'échantillon ;
- un capteur d'image, configuré pour acquérir une image de l'échantillon lorsque l'échantillon est disposé sur le support d'échantillon ;
- un processeur, programmé pour exécuter des instructions permettant de mettre en œuvre les étapes i) à vi) d'un procédé selon le premier objet de l'invention à partir d'une image acquise par le capteur d'image.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

La figure 1A représente un dispositif permettant une mise en oeuvre de l'invention
La figure 1B représente un exemple de source de lumière pouvant être utilisée dans le dispositif de la figure 1A.
La figure 2 montre l'évolution d'une onde lumineuse et illustre le retard induit par une différence de trajet optique. Le retard génère un déphasage de l'onde lumineuse.
La figure 3 représente les principales étapes d'un mode de réalisation de l'invention
Les figures 4A à 4E illustrent un exemple de mise en œuvre de l'invention.
La figure 5 montre un autre dispositif permettant une mise en œuvre de l'invention.

## EXPOSE DE MODES DE REALISATION PARTICULIERS

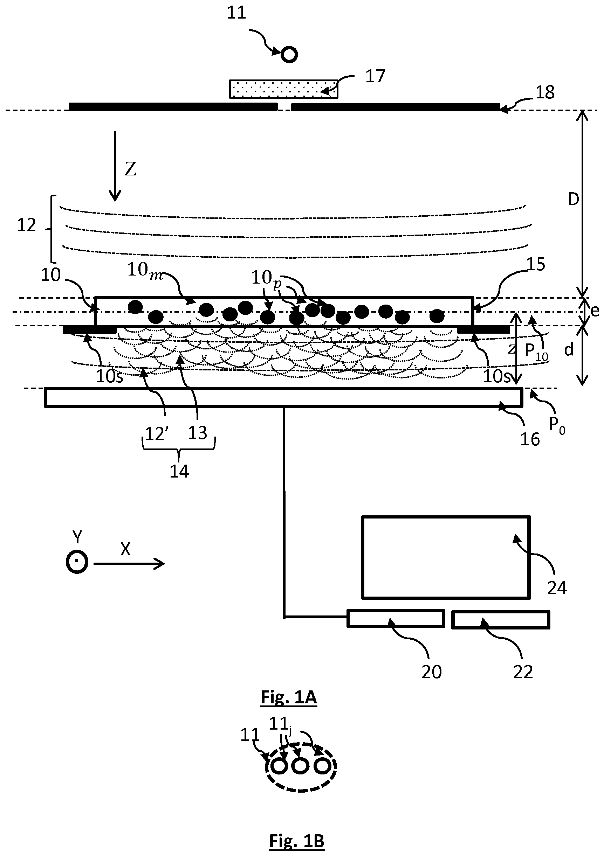

La figure 1A représente un exemple de dispositif selon l'invention. Une source de lumière 11 est apte à émettre une onde lumineuse 12, dite onde lumineuse incidente, se propageant en direction d'un échantillon 10, selon un axe de propagation Z. L'onde lumineuse est émise selon une bande spectrale $\Delta\lambda$, comportant une longueur d'onde $\lambda$. Cette longueur d'onde peut être une longueur d'onde centrale de ladite bande spectrale.

Selon un mode de réalisation, la source de lumière 11 comporte des sources de lumières élémentaires $11_j$. L'indice j désigne l'indice de la source de lumière élémentaire. Chaque source de lumière émet une onde lumineuse, selon l'axe de propagation Z, dans une bande spectrale $\Delta\lambda_j$. On peut par exemple utiliser une source de lumière 11 comportant trois sources de lumière élémentaires $11_1, 11_2, 11_3$ comme représenté sur la figure 1B. Les sources élémentaires émettent dans des bandes spectrales différentes les unes des autres, par exemple dans le bleu, dans le vert et dans le rouge. Le recours à une telle source de lumière a été décrit dans WO2016189257.

L'échantillon 10 est un échantillon que l'on souhaite caractériser. Il peut notamment s'agir d'un milieu 10m comportant des particules 10p. Les particules 10p peuvent être des particules sanguines, par exemple des globules rouges. Il peut également s'agir de cellules, de microorganismes, par exemple des bactéries ou des levures, des microalgues, des microbilles, ou des gouttelettes insolubles dans le milieu liquide, par exemple des nanoparticules lipidiques. De préférence, les particules 10p ont un diamètre, ou sont inscrites dans un diamètre, inférieur à 1 mm, et de préférence inférieur à 100 $\mu$m. Il s'agit de microparticules (diamètre inférieur à 1 mm) ou de nanoparticules (diamètre inférieur à 1 $\mu$m). Le milieu 10m, dans lequel baignent les particules, peut être un milieu liquide, par exemple une phase liquide d'un liquide corporel, d'un milieu de culture ou d'un liquide prélevé dans l'environnement ou dans un procédé industriel. Il peut également s'agir d'un milieu solide ou ayant la consistance d'un gel, par exemple un substrat de type gélose, propice à la croissance de colonies bactériennes.

L'échantillon peut également être un échantillon solide, par exemple une fine lame de tissu biologique, de type lame d'anatomopathologie, ou un extrait sec d'un fluide, par exemple d'un fluide biologique.

L'échantillon est de préférence transparent ou suffisamment translucide pour pouvoir permettre la formation d'une image par le capteur d'image.

Dans cet exemple, l'échantillon 10 est contenu dans une chambre fluidique 15. La chambre fluidique 15 est par exemple une micro-cuvette, d'utilisation courante dans les dispositifs de type point of care, dans laquelle l'échantillon 10 pénètre, par exemple par capillarité. L'épaisseur e de l'échantillon 10, selon l'axe de propagation varie typiquement entre 20 $\mu$m et 1 cm, et est de préférence comprise entre 50 $\mu$m et 500 $\mu$m, par exemple 150 $\mu$m.

L'échantillon s'étend selon un plan $P_{10}$, dit plan de l'échantillon, perpendiculaire à l'axe de propagation. Il est maintenu sur un support 10s. Le plan de l'échantillon est repéré par deux axes orthogonaux X et Y, définissant respectivement des coordonnées $x$ et $y$. Chaque couple de coordonnée ($x,y$) correspond à une coordonnée radiale r. Les coordonnées radiales sont définies dans le plan de l'échantillon et dans un plan de détection décrit ci-après.

La distance D entre la source de lumière 11 et l'échantillon 10 est de préférence supérieure à 1 cm. Elle est de préférence comprise entre 2 et 30 cm. De préférence, la source de lumière, vue par l'échantillon, est considérée comme ponctuelle. Cela signifie que son diamètre (ou sa diagonale) est préférentiellement inférieur au dixième, mieux au centième de la distance entre l'échantillon et la source de lumière. Ainsi, de préférence, la lumière parvient à l'échantillon sous la forme d'ondes planes, ou pouvant être considérées comme telles.

La source de lumière 11 peut être une diode électroluminescente ou une diode laser. Elle peut être associée à diaphragme 18, ou filtre spatial. L'ouverture du diaphragme est typiquement comprise entre 5 $\mu$m et 1 mm, de préférence entre 50 $\mu$m et 500 $\mu$m. Dans cet exemple, le diaphragme est fourni par Thorlabs sous la référence P150S et son diamètre est de 150 $\mu$m. Le diaphragme peut être remplacé par une fibre optique, dont une première extrémité est placée face à la source de lumière 11 et dont une deuxième extrémité est placée face à l'échantillon 10.

Le dispositif comporte de préférence un diffuseur 17, disposé entre la source de lumière 11 et le diaphragme 18. L'usage d'un tel diffuseur permet de s'affranchir de contraintes de centrage de la source de lumière 11 par rapport à l'ouverture du diaphragme 18. La fonction d'un tel diffuseur est de répartir le faisceau lumineux, produit par la source de lumière élémentaire 11 selon un cône d'angle $\alpha$, $\alpha$ étant égal à 30° dans le cas présent. De préférence, l'angle de diffusion $\alpha$ varie entre 10° et 80°.

De préférence, la bande spectrale d'émission $\Delta\lambda$ de l'onde lumineuse incidente 12 a une largeur inférieure à 100 nm. Par largeur de bande spectrale, on entend une largeur à mi-hauteur de ladite bande spectrale. Dans la suite du texte, chaque bande spectrale est désignée par une longueur d'onde $\lambda$ représentative de la bande spectrale, par exemple la longueur d'onde centrale.

L'échantillon 10 est disposé entre la source de lumière 11 et un capteur d'image 16. Ce dernier s'étend de préférence parallèlement, ou sensiblement parallèlement au plan selon lequel s'étend l'échantillon. Le terme sensiblement parallèlement signifie que les deux éléments peuvent ne pas être rigoureusement parallèles, une tolérance angulaire de quelques degrés, inférieure à 20° ou 10° étant admise.

Le capteur d'image 16 est apte à former une image selon un plan de détection $P_0$. Dans l'exemple représenté, il s'agit d'un capteur d'image comportant une matrice de pixels, de type CCD ou un CMOS. Les CMOS sont les capteurs préférés car la taille des pixels est plus faible, ce qui permet d'acquérir des images dont la résolution spatiale est plus favorable. Le plan de détection $P_0$ s'étend de préférence perpendiculairement à l'axe de propagation Z de l'onde lumineuse incidente 12. Ainsi, le plan de détection $P_0$ est parallèle au plan de l'échantillon $P_{10}$. Le capteur d'image comporte des pixels, à chaque pixel étant associé une coordonnée radiale r.

La distance $d$ entre l'échantillon 10 et la matrice de pixels du capteur d'image 16 est préférentiellement comprise entre 50 $\mu$m et 2 cm, de préférence comprise entre 100 $\mu$m et 2 mm.

Sur le dispositif représenté sur la figure 1A, on remarque l'absence d'optique de grossissement ou de formation d'image entre le capteur d'image 16 et l'échantillon 10. Cela n'empêche pas la présence éventuelle de microlentilles de focalisation au niveau de chaque pixel du capteur d'image 16, ces dernières n'ayant pas de fonction de grandissement de l'image acquise par le capteur d'image.

Sous l'effet de l'onde lumineuse incidente 12, l'échantillon 10 peut engendrer une onde diffractée, susceptible de produire, au niveau du plan de détection $P_0$, des interférences, en particulier avec une partie de l'onde lumineuse incidente 12 transmise par l'échantillon. Par ailleurs, l'échantillon peut absorber une partie de l'onde lumineuse incidente 12. Ainsi, l'onde lumineuse 14, transmise par l'échantillon, et à laquelle est exposé le capteur d'image 16, est formée suite à l'absorption et la diffraction de l'onde lumineuse incidente 12 par l'échantillon. Ainsi, l'échantillon entraîne une absorption d'une partie de l'onde lumineuse incidente, ainsi qu'un déphasage de cette dernière. Le déphasage est dû à une variation d'indice de réfraction (ou indice optique) lorsque l'onde lumineuse 14 se propage à travers l'échantillon.

L'onde lumineuse 14 peut également être désignée par le terme onde lumineuse d'exposition. Un processeur 20, par exemple un microprocesseur, est apte à traiter chaque image acquise par le capteur d'image 16. En particulier, le processeur est un microprocesseur relié à une mémoire programmable 22 dans laquelle est stockée une séquence d'instructions pour effectuer les opérations de traitement d'images et de calculs décrites dans cette description. Le processeur peut être couplé à un écran 24 permettant l'affichage d'images acquises par le capteur d'image 16 ou calculées par le processeur 20.

L'image acquise par le capteur d'image forme un hologramme. Elle ne permet généralement pas une représentation visuelle satisfaisante de l'échantillon, en particulier lorsque l'échantillon comporte des éléments diffractants rapprochés les uns des autres. C'est notamment le cas lorsque l'échantillon comporte des particules rapprochées les unes des autres, ou lorsque l'échantillon est une fine lame de tissu biologique. Afin d'obtenir une observation satisfaisante de l'échantillon, des algorithmes itératifs de reconstruction d'image ont été développés, tels que ceux décrits dans l'art antérieur. Ces algorithmes comportent une application itérative d'un opérateur de propagation holographique, de façon à propager l'hologramme, formé dans le plan de détection, dans un plan de reconstruction, ce dernier correspondant généralement un plan de l'échantillon, selon lequel s'étend l'échantillon. Le plan de l'échantillon est généralement parallèle au plan de détection. Les algorithmes décrits dans l'art antérieur comportent des successions de propagation / rétropropagation, d'images entre le plan de détection et le plan de l'échantillon. L'image acquise par le capteur d'image ne comporte pas d'information relative à la phase de l'onde lumineuse d'exposition. L'objectif de ces algorithmes est d'estimer, de façon itérative, la phase de l'onde lumineuse d'exposition dans le plan de détection. Cela permet de former une image correcte de l'échantillon, dans le plan de reconstruction. Ainsi, ces algorithmes permettent d'obtenir des propriétés optiques de l'onde lumineuse d'exposition 14. Il peut s'agir par exemple du module ou de la phase.

L'approche proposée par l'inventeur est différente, puisqu'elle vise non pas à obtenir des propriétés de l'onde lumineuse d'exposition 14, mais des propriétés de l'échantillon lui-même, et notamment des propriétés optiques de ce dernier. Pour cela, l'échantillon est décrit par des vecteurs $F(r)$, chaque vecteur étant défini à une coordonnée radiale $r$ dans le plan de l'échantillon. Chaque terme de chaque vecteur correspond à une propriété optique de l'échantillon.

L'inventeur a considéré qu'une propriété importante à considérer est une différence de trajet optique induite par l'échantillon, selon l'axe de propagation Z. Pour cela, on considère les coordonnées radiales $r = (x,y)$, définies dans le plan de l'échantillon. On définit, à chaque coordonnée radiale $r$, un vecteur de paramètres (ou jeu de paramètres) $F(r)$, décrivant l'échantillon. Le vecteur de paramètres $F(r)$ a une dimension $(1, N_w)$, où $N_w$ désigne le nombre de paramètres considérés. Chaque composante du vecteur de paramètres est un paramètre optique de l'échantillon.

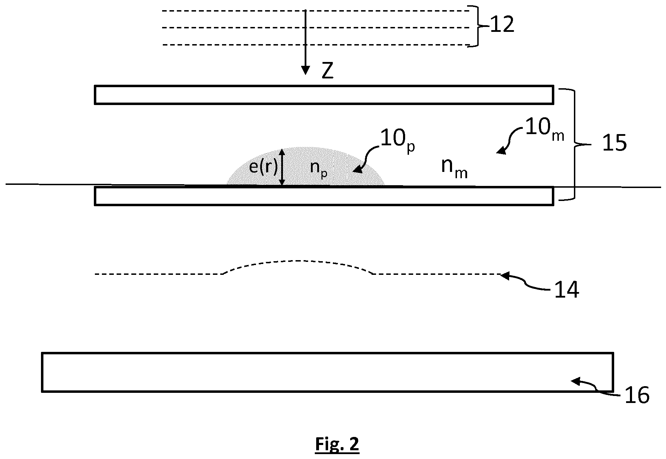

Une composante du vecteur est une différence de trajet optique $L(r)$ induit par l'échantillon selon une direction parallèle à l'axe de propagation Z. La figure 2 montre un détail d'une particule $10_p$ de l'échantillon. Dans cet exemple, la particule $10_p$ est une cellule adhérente. Elle adhère à une paroi transparente de la chambre fluidique 15. La particule $10_p$ a un indice de réfraction $n_p$, tandis que le milieu $10_m$ dans lequel elle baigne a un indice de réfraction $n_m$.

En chaque coordonnée radiale r, la différence de trajet optique $L(r)$ produite par la particule est telle que $L(r) = (n_p - n_m) \times e(r)$ où $e(r)$ : épaisseur de la particule à la coordonnée radiale $r$ et $\times$ est l'opérateur multiplication.

Sur la figure 2, on a représenté, par des pointillés, le front d'onde de l'onde lumineuse 12, incidente à l'échantillon, ainsi que de l'onde lumineuse d'exposition 14, à laquelle est exposé le capteur d'image 16, l'onde lumineuse d'exposition se propageant de l'échantillon vers le capteur d'image. Dans cet exemple, $n_p > n_m$. Le front d'onde est plan avant d'atteindre l'échantillon. En aval de l'échantillon, le front d'onde est déformé du fait de l'apparition de différences de trajet optique induites par la particule.

Une autre composante du vecteur $F(r)$ peut être une absorbance $\alpha(r)$ de l'échantillon. Lorsque la source de lumière 11 comporte différentes sources de lumière élémentaires $11_j$, le trajet optique et/ou l'absorbance peuvent être exprimés dans chaque longueur d'onde $\lambda$.

Soit $A_{10}$, l'amplitude complexe de l'onde lumineuse d'exposition 14 dans le plan de l'échantillon $P_{10}$. A chaque coordonnée radiale r, l'amplitude complexe $A_{10}(r)$ peut être définie à partir du vecteur de paramètres $F(r)$, en fonction d'hypothèses établies a priori :

- soit l'échantillon est considéré comme transparent, auquel cas l'absorbance $\alpha$ est nulle et

$$A_{10}^{\lambda}(r) = b^{\lambda}(r)exp\left(2i\pi\frac{L(r)}{\lambda}\right) (1)$$

. L'index $\lambda$ désigne une longueur d'onde et $i^2 = -1$ Comme précédemment indiqué, $\lambda$ est la longueur d'onde centrale d'une bande spectrale d'émission $\Delta\lambda$. Le terme $b^{\lambda}$ est une amplitude représentative de l'onde lumineuse incidente 12 atteignant l'échantillon. Cette amplitude peut être mesurée par le capteur d'image 16 en l'absence d'échantillon 10 sur le support 10s. La source de lumière 11 éclaire alors directement le capteur d'image. A partir de l'image $I^\lambda_{0,b}(r)$ acquise par le capteur d'image 16 dans la bande spectrale d'émission

$$b^\lambda(r) = \sqrt{I^\lambda_{0,b}(r)}. \ (1')$$

$\Delta\lambda$, on obtient l'amplitude $b^\lambda$ par

- soit l'échantillon présente une absorbance non nulle, auquel cas :

$$A^\lambda_{10}(r) = b^\lambda(r)exp(2i\pi\frac{L(r)}{\lambda} + \alpha^\lambda(r)) \ (2)$$

, où $\alpha^\lambda(r)$ est l'absorbance dans la bande spectrale $\lambda$ à la coordonnée radiale r.

On peut considérer que la différence de trajet optique $L(r)$ dépend de la bande spectrale d'émission, auquel cas elle est notée $L^\lambda(r)$. Dans ce cas, l'amplitude complexe dans le plan de l'échantillon s'écrit :

$$A^\lambda_{10}(r) = b^\lambda(r)exp(2i\pi\frac{L^\lambda(r)}{\lambda}) \ (3) \quad \text{ou} \quad A^\lambda_{10}(r) = b^\lambda(r)exp(2i\pi\frac{L^\lambda(r)}{\lambda} + \alpha^\lambda(r)) \ (4)$$ lorsque l'absorbance est respectivement considérée comme nulle ou non nulle. Selon une configuration, on définit une variable d'ajustement, définissant un saut de phase. En effet, la phase d'une onde lumineuse est définie modulo $2\pi$. On peut définir un entier $N_\varphi$ permettant d'obtenir une valeur quantitative de la phase, de telle sorte que :

$$A^\lambda_{10}(r) = b^\lambda(r)exp(2i\pi\frac{L(r)-N_\varphi(1-\frac{\lambda}{\lambda_0})}{\lambda} + \alpha^\lambda(r)) \ (5).$$

Où $\lambda_0$ désigne une longueur d'onde centrale d'une des bandes spectrales utilisées.

Chaque vecteur $F(r)$ comporte $N_w$ termes $F_w(r)$ avec $1 \leq w \leq N_w$. En fonction de l'expression de l'amplitude prise en compte, le vecteur de paramètres $F(r)$ peut varier :

- Lorsqu'on considère l'expression (1), $F(r)$ peut ne comprendre que $L(r)$.
- Lorsqu'on considère l'expression (2), $F(r)$ peut comprendre $L(r)$ et l'absorbance $\alpha^\lambda(r)$ dans chaque bande spectrale d'illumination. Lorsqu'on dispose de trois bandes spectrales d'illumination, $F(r)$ comporte $N_w = 4$ termes.
- Lorsqu'on considère l'expression (3), $F(r)$ peut comprendre $L^\lambda(r)$ dans chaque bande spectrale d'illumination. Lorsqu'on dispose de trois bandes spectrales d'illumination, $F(r)$ comporte $N_w = 3$ termes.
- Lorsqu'on considère l'expression (4), $F(r)$ peut comprendre $L^\lambda(r)$ et l'absorbance $\alpha^\lambda(r)$ dans chaque bande spectrale d'illumination. Lorsqu'on dispose de trois bandes spectrales d'illumination, $F(r)$ comporte $N_w = 6$ termes.
- Lorsqu'on considère l'expression (5), $F(r)$ peut comprendre $L(r)$ et l'absorbance $\alpha^\lambda(r)$ dans chaque bande spectrale d'illumination ainsi que l'entier $N_\varphi$ $F(r)$ comporte $N_w = 5$ termes.

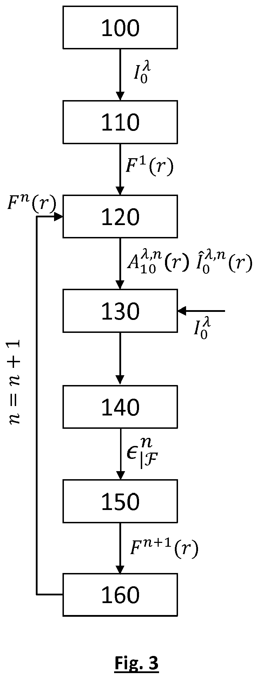

On décrit à présent, en lien avec la figure 3, un procédé permettant d'estimer le vecteur $F(r)$, tel que précédemment défini, en différentes coordonnées radiales $r$, à partir de l'image $I^\lambda_0$ acquise par le capteur d'image 16, dans une bande spectrale $\Delta\lambda$.

Etape 100 : Acquisition d'une image $I^\lambda_0$ dans chaque bande spectrale $\Delta\lambda$.

Etape 110 : Initialisation du vecteur de paramètres $F(r)$. Les termes composant le vecteur initial sont définies de façon arbitraire ou en fonction d'un a priori sur l'échantillon. Cette étape est effectuée pour chaque coordonnée radiale $r$ considérée. Les vecteurs de paramètres $F(r)$ définis lors de cette étape forment un ensemble $\mathcal{F}^1$ de vecteurs décrivant l'échantillon 10. Chaque vecteur initialisé est noté $F^1(r)$.

Les étapes 120 à 160 décrites ci-après sont mises en œuvre de façon itérative, le rang d'itération étant noté $n$. A chaque étape est associé un ensemble $\mathcal{F}^n$ de vecteurs $F^n(r)$.

Etape 120 : Pour chaque coordonnée radiale $r$, à partir du vecteur de paramètres résultant de l'étape 110, ou de l'étape 150 d'une itération précédente, détermination d'une amplitude complexe $A^{\lambda,n}_{10}(r)$ de l'onde lumineuse d'exposition 14, dans le plan de l'échantillon. L'amplitude complexe peut par exemple être déterminée à partir d'une des expressions (1) à (5). Chaque amplitude complexe $A^{\lambda,n}_{10}(r)$ forme à une image complexe $A^{\lambda,n}_{10}$ de l'échantillon. A partir de l'image complexe $A^{\lambda,n}_{10}$ ainsi déterminée, application d'un opérateur de propagation holographique $h^\lambda_{P_{10} \to P_0}$,

de façon à obtenir une image complexe $A_{10}^{\lambda,n}$ de l'onde lumineuse d'exposition 14, dans le plan de détection, selon l'expression : $A_0^{\lambda,n} = A_{10}^{\lambda,n} * h_{P_{10}\to P_0}^{\lambda}$ (6) . $h_{P_{10}\to P_0}^{\lambda}$ est un opérateur de propagation holographique, permettant une propagation du plan de l'échantillon $P_{10}$ vers le plan de détection $P_0$, à la longueur d'onde $\lambda$. Il peut s'agir d'un opérateur de Fresnel, par exemple $h(x,y,z) = \frac{1}{i\lambda z}e^{j2\pi\frac{z}{\lambda}}\exp(i\pi\frac{x^2+y^2}{\lambda z})$ (7) , avec $r = (x,y)$. D'une façon générale, l'opérateur de propagation holographique modèlise un transport de l'onde lumineuse d'exposition entre au moins deux points distants l'un de l'autre. Dans l'application décrite, le produit de convolution décrit en lien avec l'équation (6) modélise un transport de l'onde lumineuse d'exposition 14 entre le plan de l'échantillon $P_{10}$ et le plan de détection $P_0$.

En considérant la racine carrée du module de l'onde lumineuse d'exposition 14, on obtient une estimation $\hat{I}_0^{\lambda,n}$

de l'image acquise par le capteur d'image $I_0^{\lambda}$. Ainsi, $\hat{I}_0^{\lambda,n} = \sqrt{mod\,(A_0^{\lambda,n})}$ (8) où $mod$ désigne l'opérateur module. L'étape 120 est mise en œuvre dans chaque bande spectrale.

Etape 130 : Dans chaque bande spectrale, comparaison de l'image $\hat{I}_0^{\lambda}$ estimée lors de l'étape 120 avec l'image $I_0^{\lambda}$ acquise par le capteur d'image lors de l'étape 100. La comparaison peut être exprimée sous forme d'une différence ou d'un ratio, ou d'un écart quadratique.

Etape 140 : Calcul d'un indicateur de validité à partir de la comparaison effectuée, lors de l'étape 130, dans chaque bande spectrale. L'indicateur de validité $\epsilon_{|\mathcal{F}^n}^n$ représente la pertinence de l'ensemble $\mathcal{F}^n$ des vecteurs $F^n(r)$ décrivant l'échantillon. L'indice $|\mathcal{F}^n$ signifie que l'indicateur de validité est établi sachant l'ensemble $\mathcal{F}^n$ des vecteurs $F^n(r)$. Dans cet exemple, l'indicateur de validité est d'autant plus faible que l'échantillon est correctement décrit par l'ensemble $\mathcal{F}$. L'indicateur de validité $\epsilon_{|\mathcal{F}^n}^n$ comporte un critère d'erreur $\epsilon_{0|\mathcal{F}^n}^n$, ce dernier quantifiant une erreur globale de l'image estimée $\hat{I}_0^{\lambda,n}$ par rapport à l'image mesurée $I_0^{\lambda}$. Par erreur globale, on entend une erreur pour chaque coordonnée radiale et pour chaque bande spectrale considérée.

Le critère d'erreur $\epsilon_{0|\mathcal{F}^n}^n$ est établi à partir de la comparaison des images $\hat{I}_0^{\lambda,n}$ et $I_0^{\lambda}$. Par exemple,

$$\epsilon_{0|\mathcal{F}^n}^n = \frac{1}{N_r N_\lambda}\int dr \sum_\lambda \left(\frac{I_0^{\lambda}(r)-\hat{I}_0^{\lambda,n}(r)}{\sigma\left(I_0^{\lambda,n}(r)\right)}\right)^2 \text{ (10)},$$

où :

- $N_r$ est le nombre de coordonnées radiales considérées ;
- $N_\lambda$ est le nombre de bandes spectrales considérées ;
- $\sigma$ est l'opérateur écart-type, moyennant une prise en compte d'un modèle de bruit.

L'indice $0|\mathcal{F}^n$ attribué au critère d'erreur $\epsilon_{0|\mathcal{F}^n}^n$ représente le fait que cet indicateur est calculé dans le plan de détection, sachant l'ensemble des vecteurs $\mathcal{F}^n$ pris en compte lors de l'itération. Le critère d'erreur $\epsilon_{0|\mathcal{F}^n}^n$ est un critère d'attache aux données, dans le sens ou sa minimisation permet de se rapprocher des données mesurées, en l'occurrence chaque image $I_0^{\lambda}$. Aussi, lorsque $\hat{I}_0^{\lambda,n}$ tend vers $I_0^{\lambda}$, c'est-à-dire lorsque l'ensemble $\mathcal{F}^n$ des vecteurs décrivent correctement l'échantillon 10, $\epsilon_{0|\mathcal{F}^n}^n$ tend vers 1. En effet, compte tenu du bruit, le terme $\left(\frac{I_0^{\lambda}(r)-\hat{I}_0^{\lambda,n}(r)}{\sigma\left(I_0^{\lambda,n}(r)\right)}\right)^2$

tend vers 1.

En prenant en compte la transformée de Anscombe, en considérant que l'image $I_0^\lambda$ est affectée d'un bruit poissonnien, on a $\widehat{M}_0^{\lambda,n} = \sqrt{\hat{I}_0^{\lambda,n} + \frac{3}{8}}\,(11)$ et $M_0^\lambda = \sqrt{I_0^\lambda + \frac{3}{8}}\,(11')$ où $\widehat{M}_0^{\lambda,n}$ et $M_0^\lambda$ correspondent respectivement aux modules des images $\hat{I}_0^{\lambda,n}$ et $I_0^\lambda$.

L'expression (10) devient alors : $\epsilon_{0|\mathcal{F}^n}^n = \frac{4}{N_r N_\lambda} \int dr \sum_\lambda \left(\widehat{M}_0^{\lambda,n} - M_0^\lambda\right)^2 (12)$ car du fait de cette transformation, l'écart type est transformé en un écart type constant de valeur ½.

Selon un mode de réalisation, $\epsilon_{|\mathcal{F}^n}^n = \epsilon_{0|\mathcal{F}^n}^n\,(13)$ : l'indicateur de validité ne prend en compte que le critère d'erreur.
Dans un autre mode de réalisation, détaillé par la suite, l'indicateur de validité comporte également un critère morphologique, correspondant à la prise en compte de contraintes géométriques ou optiques de l'échantillon ou des particules formant l'échantillon.

Etape 150 : Mise à jour des vecteurs $F^n(r)$ par minimisation de l'indicateur de validité $\epsilon_{|\mathcal{F}^n}^n$. L'indicateur de validité $\epsilon_{|\mathcal{F}^n}^n$ est une variable scalaire. Cependant, il dépend de l'ensemble $\mathcal{F}^n$ des vecteurs paramètres sur la base desquels il a été établi, par l'intermédiaire de l'image $\hat{I}_0^{\lambda,n}$ estimée dans chaque bande spectrale considérée.
Au cours de l'étape 150, on applique un algorithme de minimisation, de type descente de gradient, de façon à s'approcher progressivement, à chaque itération, de l'ensemble $\mathcal{F}^n$ permettant une minimisation satisfaisante de l'indicateur de validité $\epsilon_{|\mathcal{F}^n}^n$. Aussi, l'objectif de cette étape est d'établir un ensemble $\mathcal{F}^{n+1}$ de vecteurs $F^{n+1}(r)$ visant à obtenir, suite à une réitération des étapes 110 à 140, un indicateur de validité $\epsilon_{|\mathcal{F}^{n+1}}^{n+1}$ plus faible que l'indicateur de validité $\epsilon_{|\mathcal{F}^n}^n$ de l'itération courante.

Cette étape permet de mettre à jour au moins un terme $F_w^n(r)$ de chaque vecteur $F^n(r)$.

Pour cela, on définit un gradient $G_w^n(r)$ de l'indicateur de validité $\epsilon_{|\mathcal{F}^n}^n$ à l'égard du paramètre optique correspondant au terme $F_w^n(r)$, de telle sorte que :

$$G_w^n(r) = \frac{\partial \epsilon_{|\mathcal{F}^n}^n}{\partial F_w^n(r)}\,(14)$$

Un algorithme de descente de gradient définit alors une direction $d_w^n$ ainsi qu'un pas d'avancement $\sigma_w^n$. Le terme $F_w(r)$ de chaque vecteur de paramètre est mis à jour selon l'expression : $F_w^{n+1}(r) = F_w^n(r) + d_w^n\,\sigma_w^n\,(15)$

Le gradient $G_w^n(r)$ peut être défini pour chaque terme $F_w^n(r)$ des vecteurs $F^n(r)$.
Etape 160 : réitération des étapes 120 à 150, en prenant en compte, lors de l'étape 120 de l'itération suivante, l'ensemble $\mathcal{F}^{n+1}$ mis à jour lors de l'étape 150 de l'itération dernièrement effectuée.

Les étapes 120 à 160 sont réitérées jusqu'à ce que la valeur de l'indicateur de validité $\epsilon_{|\mathcal{F}^n}^n$ soit considérée comme représentative d'une bonne description de l'échantillon par l'ensemble $\mathcal{F}^n$ de vecteurs $F^n(r)$. En prenant en compte un indicateur tel que défini dans les équations (10) et (13), les itérations cessent lorsque la valeur de l'indicateur

de validité $\epsilon_{|\mathcal{F}^n}^n$ est suffisamment faible.

Selon une variante, lors de l'étape 140, l'indicateur de validité $\epsilon_{|\mathcal{F}^n}^n$ prend également en compte un critère morphologique $\epsilon_{10|\mathcal{F}^n}^n$.

A la différence du critère d'erreur $\epsilon_{0|\mathcal{F}^n}^n$, qui est défini à partir de données mesurées ou estimées dans le plan de détection $P_0$, le critère morphologique $\epsilon_{10|\mathcal{F}^n}^n$ est défini dans le plan de l'échantillon $P_{10}$.

D'une façon générale, le critère morphologique $\epsilon_{10|\mathcal{F}^n}^n$ dépend de la valeur des termes des vecteurs de paramètres déterminés lors de l'étape 110 ou lors de l'étape 150 d'une itération précédente, ou de leur dérivées spatiales. Il est représentatif de la morphologie de l'échantillon, telle que déterminée à partir des vecteurs de paramètres. Autrement dit, le critère morphologique représente un critère d'attache à des données morphologiques de l'échantillon, ces dernières pouvant être définies par des hypothèses.

Le critère morphologique $\epsilon_{10|\mathcal{F}^n}^n$ peut prendre en compte une dérivée spatiale de la différence de trajet optique, de façon à prendre en compte une forme prédéfinie d'une particule. Par exemple, lorsque l'échantillon comporte des cellules adhérentes, la forme prédéfinie peut être un hémisphère, un tel cas de figure étant représenté sur la figure 2. Lorsque l'échantillon comporte des cellules flottantes, la forme prédéfinie peut être une sphère lorsque les cellules sont sphériques.

D'une façon générale le critère morphologique peut être exprimé comme suit :

$$\epsilon_{10|\mathcal{F}^n}^n = \int d\vec{r} \sum_k a_k \left( \sum_l b_{kl} . \left| O_{kl} * \sum_\lambda f_{kl} \left( F_{kl}^{\lambda,n}(r) \right) \right|^2 \right)^{n_k} \quad (16)$$

- $a_k$ et $b_{kl}$ sont des réels, formant des facteurs de pondération ;
- $O_{kl}$ est un opérateur, par exemple un opérateur de type dérivée partielle spatiale, ou racine carrée, ou le laplacien.
- $F_{kl}^{\lambda,n}(r)$ désigne un terme d'un vecteur $F^n(r)$, auquel est associé le terme de pondération $b_{kl}$, le terme étant défini à la longueur d'onde $\lambda$.
- $f_{kl}$ désigne une fonction appliquée au terme $F_{kl}^{\lambda,n}(r)$, par exemple une fonction trigonométrique, de type sinus ou cosinus, mais il peut également s'agir de la fonction identité, telle que $f_{kl}\left( F_{kl}^{\lambda,n}(r) \right) = F_{kl}^{\lambda,n}(r)$ ;
- $n_k$ est un réel positif, par exemple égal à 0.5 lorsqu'on souhaite utiliser une norme d'ordre 1, ou égal à 1 lorsqu'on souhaite utiliser une norme d'ordre 2 ;
- $*$ désigne, dans cette expression, l'application de l'opérateur $O_{kl}$.

Le critère morphologique est donc, dans sa forme la plus générale, une somme pondérée d'opérateurs que l'on applique à différents termes de chaque vecteur $F^n(r)$. Cette somme pondérée est intégrée selon les différentes coordonnées radiales considérées, de façon à former un indicateur morphologique $\epsilon_{10|\mathcal{F}^n}^n$ prenant la forme d'une variable scalaire.

Par exemple, si l'amplitude complexe de l'onde lumineuse d'exposition 14 est définie selon l'expression (1), chaque vecteur de paramètre comporte un terme $L^n(r)$ un exemple de critère morphologique est :

$$\epsilon_{10|\mathcal{F}^n}^n = \int dr \sqrt{\left( \frac{\partial L^n(r)}{\partial x} \right)^2 + \left( \frac{\partial L^n(r)}{\partial y} \right)^2} \quad (16)'$$

Ce critère tend à diminuer lorsque la grandeur $L^n(r)$ ne présente un minium d'oscillations, ce qui est par exemple le cas lorsque les particules ont une morphologie sphérique ou hémisphérique de particules. Les valeurs de $L^n(r)$ pour lesquelles le critère est minimal correspondent donc à des particules, par exemple sphériques ou hémisphériques, isolées les unes des autres, avec un minimum d'oscillation de $L^n(r)$ entre les particules ou sur ces dernières.

Le critère morphologique $\epsilon_{10|\mathcal{F}^n}^n$ est minimal lorsque les vecteurs de paramètres $F^n(r)$ formant l'ensemble $\mathcal{F}^n$ décrivent des objets répondant à des hypothèses morphologiques établies à priori.

Lorsque l'indicateur de validité $\epsilon_{|\mathcal{F}^n}$ inclut une prise en compte du critère morphologique $\epsilon_{10|\mathcal{F}^n}^n$, il peut être défini sous la forme d'une somme pondérée du critère d'erreur $\epsilon_{0|\mathcal{F}^n}^n$ et du critère morphologique $\epsilon_{10|\mathcal{F}^n}^n$. L'expression de l'indicateur de validité peut alors être, par exemple :

$$\epsilon_{|\mathcal{F}^n}^n = \epsilon_{0|\mathcal{F}^n}^n + \gamma\epsilon_{10|\mathcal{F}^n}^n \ (17)$$

où $\gamma$ est un scalaire positif.

La variation de $\epsilon_{|\mathcal{F}^n}^n$ pour une variation de trajet optique $\delta L^n(r)$ peut être exprimée par l'expression :

$$\delta\epsilon_{|\mathcal{F}^n}^n = 2\int dr \sum_\lambda \left(\widehat{M}_0^{\lambda,n} - M_0^\lambda\right)\delta\widehat{M}_0^{\lambda,n} + \gamma\int dr \frac{\frac{\partial L^n(r)}{\partial x}\frac{\partial L^n(r)}{\partial x} + \frac{\partial L^n(r)}{\partial y}\frac{\partial L^n(r)}{\partial y}}{\sqrt{\left(\frac{\partial L^n(r)}{\partial x}\right)^2 + \left(\frac{\partial L^n(r)}{\partial y}\right)^2}} \ (18)$$

où :

- $\widehat{M}_0^\lambda$ est le module de l'amplitude complexe $\hat{A}_0^\lambda$ estimée dans le plan de l'échantillon ;
- $\delta\widehat{M}_0^\lambda$ représente une variation du module $\delta\widehat{M}_0^\lambda$ sous l'effet d'une variation $\delta L(r)$ du trajet optique en chaque coordonnée radiale $r$.

On peut montrer que :

$$G_w^n(r) = \frac{\partial\epsilon_{0|\mathcal{F}}^n}{\partial L(r)} = 4\pi Im \sum_\lambda \left(\left(\left(B^\lambda(r)(1 - \frac{M_0^{\lambda,n}}{\widehat{M}_0^\lambda})\right)A_0^{\lambda,n}\right) * h_{P_{10}\to P_0}^\lambda \cdot \frac{A_{10}^{\lambda,n}}{\lambda} - \gamma \cdot \left(\frac{\partial}{\partial x}\frac{\left(\frac{\partial L^n(r)}{\partial x}\right)}{\epsilon_M(r)} + \right.$$

$$\left. \frac{\partial}{\partial x}\frac{\left(\frac{\partial L^n(r)}{\partial x}\right)}{\epsilon_M(r)}\right) \ (19),$$

où $Im$ désigne l'opérateur partie imaginaire. avec :

$$\epsilon_M(r) = \sqrt{\left(\frac{\partial L^n(r)}{\partial x}\right)^2 + \left(\frac{\partial L^n(r)}{\partial y}\right)^2} \ (19')$$

Le terme $B^\lambda(r)$ est déterminé lors d'une exposition du capteur d'image à une illumination de la source de lumière sans échantillon disposé entre la source de lumière et le capteur d'image.

On peut ainsi déterminer, à chaque itération, une variation $\delta L^n(r)$, en chaque coordonnée radiale r, induisant une décroissance progressive de l'indicateur de validité $\epsilon_{|\mathcal{F}^n}^n$.

Lorsqu'entre deux itérations successives, l'indicateur de validité $\epsilon_{|\mathcal{F}^n}^n$ ne décroît plus de façon significative, les itérations cessent. L'échantillon est alors considéré comme représenté, de façon correcte, par les vecteurs $F^n(r)$ formant l'ensemble $\mathcal{F}^n$. Les itérations peuvent également cesser lorsqu'un nombre prédéterminé d'itérations est atteint.

Selon un mode de réalisation, la valeur du scalaire $\gamma$ varie en fonction des itérations. Ainsi, au cours de chaque itération, on calcule un indicateur de validité tel que :

cours ae cnaque iteration, on ca

$$\epsilon_{|\mathcal{F}^n}^n = \epsilon_{0|\mathcal{F}^n}^n + \gamma^n \epsilon_{10|\mathcal{F}^n}^n \ (20)$$

$\gamma^n$ est la valeur du coefficient de pondération lors d'une itération $n$.

Au cours des premières itérations, $\gamma^n$ est nul ou proche de zéro. L'indicateur de validité privilégie alors l'attache aux données, par le biais du critère d'erreur $\epsilon_{0|\mathcal{F}^n}^n$. Lorsque le rang $n$ des itérations augmente, la valeur du terme de pondération $\gamma^n$ augmente, de façon à davantage prendre en compte le critère morphologique $\epsilon_{10|\mathcal{F}^n}^n$. Selon ce mode de réalisation, lors de premières itérations, l'indicateur de validité $\epsilon_{|\mathcal{F}^n}^n$ est uniquement calculé à partir du critère d'erreur $\epsilon_{0|\mathcal{F}^n}^n$. Puis, lorsqu'on dispose d'une détermination un peu plus précise de l'échantillon, par le biais des vecteurs $F^n(r)$, l'indicateur de validité prend en compte le critère morphologique $\epsilon_{10|\mathcal{F}^n}^n$.

Selon un mode de réalisation, les vecteurs $F^n(r)$ décrivant l'échantillon 10 évoluent. Par exemple, au cours des premières itérations des étapes 120 à 160, la différence de trajet optique est considérée comme indépendante de la longueur d'onde. Chaque vecteur $F^n(r)$ comporte donc un terme $L^n(r)$ correspondant à la différence de trajet optique. Dans un second temps, par exemple lorsque le terme $L^n(r)$ n'évolue pas de façon significative entre deux itérations successives, ou au bout d'un nombre prédéterminé d'itérations, chaque vecteur $F^n(r)$ comporte autant de termes $L^{\lambda,n}(r)$ que de bandes spectrales d'émission, ces termes étant indépendants les uns des autres. Cela permet de raffiner le modèle de l'échantillon.

Selon un mode de réalisation, lors de l'étape 120, on prend en compte des imperfections de la source de lumière. Il est par exemple possible de prendre en compte la taille de la source. Il s'agit de considérer le fait que la source n'est pas parfaitement ponctuelle. A cette fin, l'image estimée dans le plan de détection est convoluée par un noyau de convolution K traduisant une surface de la source parallèlement au plan de détection. Ainsi,

$$\hat{I}_0^{\lambda,n} = \sqrt{mod\left(A_0^{\lambda,n}\right)} * K \ (21),$$

où * est le produit de convolution.

Selon un mode de réalisation, lors de l'étape 120, on prend en compte la largeur spectrale de chaque bande spectrale d'émission $\Delta\lambda$. Ainsi, dans chaque bande spectrale, l'image $\hat{I}_0^{\lambda,n}$ est définie en prenant en compte un taux d'émission $s(d\lambda)$ dans une bande spectrale élémentaire $d\lambda$. La bande spectrale élémentaire $d\lambda$ correspond au pas de discrétisation de la bande spectrale considérée.

$$\hat{I}_0^{\lambda,n} = \int d\lambda s(d\lambda) \left| A_0^{\lambda+d\lambda,n} * h_{P_{10}\rightarrow P_0}^{\lambda+d\lambda} \right| \ (22)$$

On peut considérer que $A_0^{\lambda+d\lambda,n} = A_0^{\lambda,n}$, en négligeant les variations de $A_0$ avec $d\lambda$.

Exemple

Un exemple a été réalisé en utilisant un échantillon comportant des cellules de type A549 disposées dans un échantillon comportant un milieu de culture liquide, à une distance de 1400 $\mu$m d'un capteur d'image de type CMOS : taille des pixels : 1.67 $\mu$m x 1.67 $\mu$m - 10 millions de pixels. La source de lumière comportait 3 diodes électroluminescentes émettant respectivement dans le rouge, le vert et le bleu. La source de lumière était disposée à une distance de 50 mm de l'échantillon. Elle était couplée à un diaphragme définissant une ouverture de diamètre 50 $\mu$m.

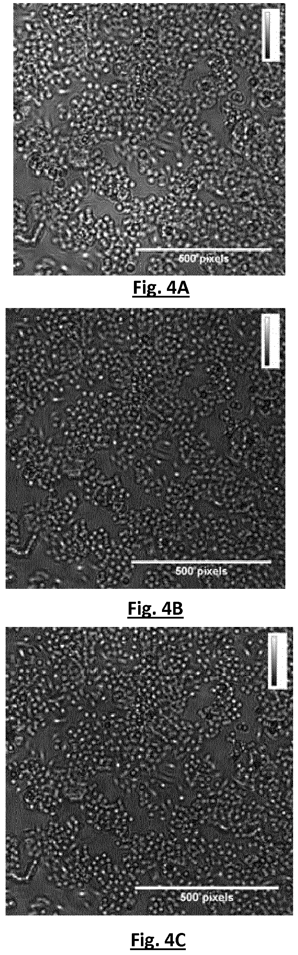

Les figures 4A, 4B et 4C montrent les images respectivement acquises dans les bandes spectrales rouges, vertes et bleues.

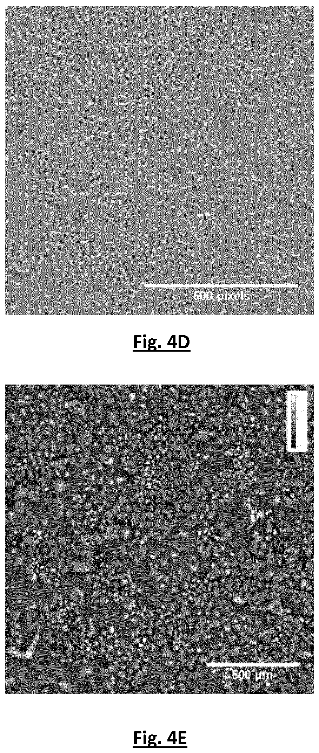

La figure 4D montre une image du gradient $G_w^n(r)$ de l'indicateur de validité $\epsilon_{|\mathcal{F}}^n$ établi pour chaque pixel r, en fonction du paramètre L(r) lors de la première itération ($n$ = 1). Dans cet exemple, l'indicateur de validité est tel que :

$$\epsilon_{|\mathcal{F}^n}^n = \epsilon_{0|\mathcal{F}^n}^n + \gamma\epsilon_{10|\mathcal{F}^n}^n \quad (24)$$

$$\text{Avec } \epsilon_{0|\mathcal{F}^n}^n = \frac{4}{N_r N_\lambda} \int dr \sum_\lambda \left(\widehat{M}_0^{\lambda,n} - M_0^\lambda\right)^2 \quad (25)$$

et

$$\epsilon_{10|\mathcal{F}^n}^n = \int dr \sqrt{\left(\frac{\partial L^n(r)}{\partial x}\right)^2 + \left(\frac{\partial L^n(r)}{\partial y}\right)^2} + \sqrt{(L^n(r))^2} \quad (26)$$

La figure 4E montre une distribution spatiale de l'épaisseur optique $L^n(r)$ dans l'échantillon à l'itération $n$ = 100. Cette figure permet de visualiser les cellules et d'obtenir, pour chacune d'entre elle, une valeur quantitative de l'épaisseur optique.

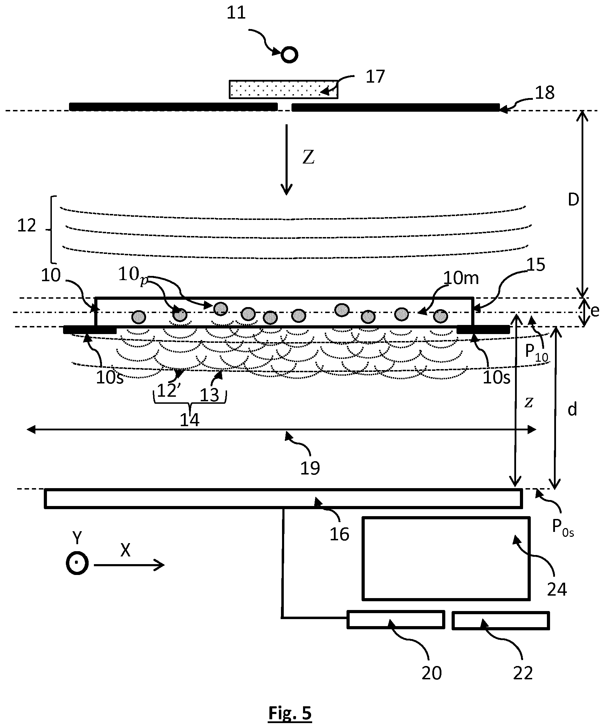

La figure 5 schématise un dispositif permettant une mise en œuvre de l'invention. Contrairement au dispositif représenté sur la figure 1A, le dispositif de la figure 5 comporte un système optique 19 de formation d'image. Le système optique 19 définit un plan image et un plan objet. Le système optique peut être une lentille ou un objectif. Lors de l'acquisition de l'image de l'échantillon, le capteur d'image est disposé selon une configuration défocalisée. Le plan de détection est décalé par rapport au plan image et/ou le plan selon lequel s'étend l'échantillon est décalé par rapport au plan objet. Le décalage est généralement faible, en étant de préférence inférieur à 1 mm, et typiquement dans une plage 50 μm - 500 μm.

L'invention pourra être mise en œuvre pour l'observation d'échantillons dans le domaine de la biologie ou de la santé, dans le domaine du contrôle de l'environnement ou dans d'autres domaines industriels, γ compris l'agroalimentaire.

## Revendications

1. Procédé d'observation d'un échantillon (10), l'échantillon s'étendant selon un plan de l'échantillon ($P_{10}$) définissant des coordonnées radiales ($r$), chaque coordonnée radiale correspondant à un couple de deux coordonnées ($x,y$) selon deux axes orthogonaux (X,Y) du plan de l'échantillon, le procédé comportant les étapes suivantes :

   a) illumination de l'échantillon à l'aide d'une source de lumière (11), configurée pour émettre une onde lumineuse incidente (12) se propageant vers l'échantillon selon un axe de propagation (Z);

   b) acquisition, à l'aide d'un capteur d'image (16), d'une image $(I_0^\lambda)$ de l'échantillon (10), formée dans un plan de détection ($P_0$), ladite image de l'échantillon formant un hologramme de l'échantillon, l'échantillon étant disposé entre la source de lumière (11) et le capteur d'image (16), de telle sorte que l'onde lumineuse incidente subit une différence de trajet optique, parallèlement à l'axe de propagation (Z), en traversant l'échantillon;
   c) traitement de l'image acquise par le capteur d'image ;

   le procédé étant tel que le traitement de l'image acquise comporte les étapes itératives suivantes:

   i) initialisation d'un ensemble $(\mathcal{F}^n)$ de vecteurs de paramètres ($F^n(r)$), modélisant l'échantillon, les vecteurs de paramètres étant respectivement définis en plusieurs coordonnées radiales ($r$), dans le plan de l'échantillon, chaque vecteur de paramètres étant associé à une coordonnée radiale ($r$), et comportant au moins une composante $\left(F_w^n(r)\right)$ représentative d'un paramètre optique de l'échantillon en ladite coordonnée radiale, au moins un paramètre optique étant une différence de trajet optique induite par l'échantillon à ladite coordonnée radiale ;

ii) à partir des vecteurs de paramètres ($F^n(r)$), formation d'une image complexe $(A_{10}^{\lambda,n})$ de l'échantillon modélisé dans le plan de l'échantillon ($P_{10}$) ;

iii) application d'un opérateur de propagation holographique à l'image complexe de l'échantillon modélisé formée dans le plan de l'échantillon, pour calculer une image $(\hat{I}_0^{\lambda,n})$ de l'échantillon modélisé dans le plan de détection ;

iv) comparaison de l'image $(I_0^{\lambda})$ acquise lors de l'étape b) et de l'image $(\hat{I}_0^{\lambda,n})$ calculée lors de l'étape iii), pour calculer un indicateur de validité $(\epsilon_{|\mathcal{F}}^n)$ ;

v) mise à jour de l'ensemble des vecteurs de paramètres, de façon à faire tendre l'indicateur de validité $(\epsilon_{|\mathcal{F}}^n)$ vers une valeur prédéterminée ;

vi) réitération des étapes ii) à v) en prenant en compte les vecteurs de paramètres ($F^{n+1}(r)$) mis à jour lors de l'étape v) ;

le procédé étant **caractérisé en ce que** :

l'étape v) comporte un calcul d'un critère morphologique $(\epsilon_{10|\mathcal{F}}^n)$ à partir des vecteurs de paramètres ($F^n(r)$), le critère morphologique quantifiant un écart des vecteurs de paramètres, décrivant l'échantillon, par rapport à une contrainte morphologique de l'échantillon, de telle sorte que l'indicateur de validité $(\epsilon_{|\mathcal{F}}^n)$ est obtenu à partir d'une combinaison :

- d'un critère d'erreur $(\epsilon_{0|\mathcal{F}}^n)$, déterminé à partir d'une comparaison entre l'image acquise lors de l'étape b) et de l'image obtenue lors de l'étape iii) ;
- et du critère morphologique $(\epsilon_{10|\mathcal{F}}^n)$.

2. Procédé selon la revendication 1, dans lequel l'étape v) comporte une détermination d'un gradient $(G_w^n(r))$ de l'indicateur de validité $(\epsilon_{|\mathcal{F}}^n)$ en fonction d'au moins un paramètre $(F_w^n(r))$, de telle sorte que les vecteurs de paramètres ($F^{n+1}(r)$) sont mis à jour pour réduire l'indicateur de validité de l'itération suivante $(\epsilon_{|\mathcal{F}}^{n+1})$.

3. Procédé selon l'une quelconque des revendications 1 ou 2, dans lequel l'étape v) met en œuvre un algorithme de type descente de gradient.

4. Procédé selon l'une quelconque des revendications précédentes, dans lequel chaque vecteur de paramètre ($F^n(r)$) comporte:

- au moins un paramètre optique ($L(r)$) représentatif d'une différence de trajet optique selon l'axe de propagation ;
- un paramètre optique ($\alpha(r)$) représentatif de l'absorbance.

5. Procédé selon l'une quelconque des revendications précédentes, dans lequel l'indicateur de validité $(\epsilon_{|\mathcal{F}}^n)$ est une somme, éventuellement pondérée, du critère d'erreur $(\epsilon_{0|\mathcal{F}}^n)$ et du critère morphologique $(\epsilon_{10|\mathcal{F}}^n)$.

6. Procédé selon la revendication 5, dans lequel l'indicateur de validité $(\epsilon_{|\mathcal{F}}^n)$ est une somme pondérée du critère d'erreur $(\epsilon_{0|\mathcal{F}}^n)$ et du critère morphologique $(\epsilon_{10|\mathcal{F}}^n)$, la pondération ($\gamma$) variant entre au moins deux itérations successives.

7. Procédé selon l'une quelconque des revendications précédentes, dans lequel :

- lors de l'étape b), une image de l'échantillon $(I_0^{\lambda})$ est acquise dans différentes bandes spectrales ($\lambda$);

- chaque vecteur de paramètres comporte au moins un paramètre optique défini dans une des bandes spectrales ;

le procédé étant tel que :

- lors de l'étape iii), l'image complexe de l'échantillon modélisé $(A_{10}^{\lambda,n})$ est propagée dans chaque bande spectrale, de façon à calculer, dans chaque bande spectrale, une image de l'échantillon modélisé $(\hat{I}_0^{\lambda,n})$ ;

- l'étape iv) comporte une comparaison, dans chaque bande spectrale $(\lambda)$, de l'image $(\hat{I}_0^{\lambda,n})$ calculée lors de l'étape iii) et de l'image $(I_0^{\lambda})$ acquise lors de l'étape b) ;

- de telle sorte que l'indicateur de validité calculé dans l'étape iv) est calculé à partir des comparaisons effectuées lors de l'étape iv).

8. Procédé selon la revendication 7, dans lequel :

- lors de premières itérations, un paramètres optique est considéré comme ne variant pas en fonction de la bande spectrale ;
- au-delà de premières itérations, le paramètre optique est considéré comme variable en fonction de la bande spectrale.

9. Procédé selon l'une quelconque des revendications précédentes, dans lequel lors de l'étape iii), le calcul de l'image de l'échantillon modélisé $(\hat{I}_0^{\lambda,n})$ dans le plan de détection $(P_0)$ comporte l'application d'un produit de convolution par un noyau de convolution $(K)$, le noyau de convolution représentant une étendue spatiale de la source de lumière.

10. Procédé selon l'une quelconque des revendications précédentes, dans lequel lors de l'étape iii), le calcul de l'image de l'échantillon modélisé $(\hat{I}_0^{\lambda,n})$ dans le plan de détection $(P_0)$ comporte une intégrale d'une image élémentaire, définie dans une bande spectrale élémentaire, l'intégrale étant calculée pour tenir compte d'une bande spectrale d'émission de la source de lumière.

11. Dispositif d'observation d'un échantillon (10) comportant :

- une source de lumière (11), configurée pour émettre une onde lumineuse incidente pour illuminer l'échantillon ;
- un support d'échantillon (10s), configuré pour recevoir l'échantillon ;
- un capteur d'image (16), configuré pour acquérir une image de l'échantillon lorsque l'échantillon est disposé sur le support d'échantillon (10s) ;
- un processeur (20), programmé pour exécuter des instructions permettant de mettre en œuvre les étapes i) à vi) d'un procédé selon l'une quelconque des revendications précédentes à partir d'une image acquise par le capteur d'image.

**Patentansprüche**

1. Verfahren zur Beobachtung einer Probe (10), wobei sich die Probe in einer Probenebene $(P_{10})$ erstreckt, die radiale Koordinaten $(r)$ definiert, wobei jede radiale Koordinate einem Paar von zwei Koordinaten $(x,y)$ in zwei orthogonalen Achsen (X, Y) der Probenebene entspricht, wobei das Verfahren die folgenden Schritte umfasst:

a) Beleuchtung der Probe mithilfe einer Lichtquelle (11), die dazu konfiguriert ist, eine einfallende Lichtwelle (12) zu emittieren, die sich in einer Fortpflanzungsachse (Z) zur Probe hin fortpflanzt;

b) Erfassung eines Bildes $(I_0^{\lambda})$ der Probe (10) mithilfe eines Bildsensors (16), das in einer Detektionsebene $(P_0)$ gebildet wird, wobei das Bild der Probe ein Hologramm der Probe bildet, wobei die Probe zwischen der Lichtquelle (11) und dem Bildsensor (16) derart angeordnet ist, dass die einfallende Lichtwelle beim Durchlaufen der Probe einer Lichtwegdifferenz parallel zur Fortpflanzungsachse (Z) unterliegt;

c) Bearbeitung des durch den Bildsensor erfassten Bildes;

wobei das Verfahren derart ist, dass die Bearbeitung des erfassten Bildes die folgenden iterativen Schritte umfasst:

i) Initialisierung eines Satzes ($F^n$) von Parametervektoren ($F^n(r)$), der die Probe modelliert, wobei die Parametervektoren jeweils als mehrere radiale Koordinaten ($r$) in der Probenebene definiert sind, wobei jeder Parametervektor einer radialen Koordinate ($r$) zugeordnet ist, und mindestens eine Komponente $\left(F_w^n(r)\right)$ umfasst, die für einen optischen Parameter der Probe an der radialen Koordinate repräsentativ ist, wobei mindestens ein optischer Parameter eine Lichtwegdifferenz ist, die durch die Probe an der radialen Koordinate herbeigeführt wird;

ii) auf der Grundlage der Parametervektoren ($F^n(r)$) Bildung eines komplexen Bildes $\left(A_{10}^{\lambda,n}\right)$ der in der Probenebene ($P_{10}$) modellierten Probe;

iii) Anwendung eines Operators zur holografischen Fortpflanzung auf das in der Probenebene gebildete komplexe Bild der modellierten Probe zur Berechnung eines Bildes $\left(\hat{I}_0^{\lambda,n}\right)$ der modellierten Probe in der Detektionsebene;

iv) Vergleich des in Schritt b) erfassten Bildes $\left(I_0^{\lambda}\right)$ und des in Schritt iii) berechneten Bildes $\left(\hat{I}_0^{\lambda,n}\right)$ zur Berechnung eines Gültigkeitsindikators $\left(\epsilon_{|F}^n\right)$;

v) Aktualisierung des Satzes von Parametervektoren, so dass der Gültigkeitsindikator $\left(\epsilon_{|F}^n\right)$ auf einen vorbestimmten Wert ausgedehnt wird;
vi) Wiederholung der Schritte ii) bis v) unter Berücksichtigung der in Schritt v) aktualisierten Parametervektoren ($F^{n+1}(r)$);

wobei das Verfahren **dadurch gekennzeichnet ist, dass**:

Schritt v) eine Berechnung eines morphologischen Kriteriums $\left(\epsilon_{10|F}^n\right)$ auf der Grundlage der Parametervektoren ($F^n(r)$) umfasst, wobei das morphologische Kriterium eine die Probe beschreibende Abweichung der Parametervektoren von einer morphologischen Vorgabe der Parametervektoren derart quantifiziert, dass der Gültigkeitsindikator $\left(\epsilon_{|F}^n\right)$ auf der Grundlage einer Kombination:

- aus einem Fehlerkriterium $\left(\epsilon_{0|F}^n\right)$, das auf der Grundlage eines Vergleichs zwischen dem in Schritt b) erfassten Bild und dem in Schritt iii) erhaltenen Bild bestimmt wird;

- und aus dem morphologischen Kriterium $\left(\epsilon_{10|F}^n\right)$

erhalten wird.

2. Verfahren nach Anspruch 1, wobei Schritt v) eine Bestimmung eines Gradienten $\left(G_w^n(r)\right)$ des Gültigkeitsindikators $\left(\epsilon_{|F}^n\right)$ in Abhängigkeit von mindestens einem Parameter $\left(F_w^n(r)\right)$ derart umfasst, dass die Parametervektoren ($F^{n+1}(r)$) aktualisiert werden, um den Gültigkeitsindikator der folgenden Iteration $\left(\epsilon_{|F}^{n+1}\right)$ zu verringern.

3. Verfahren nach einem der Ansprüche 1 oder 2, wobei Schritt v) einen Algorithmus vom Typ Gradientenabstieg durchführt.

4. Verfahren nach einem der vorhergehenden Ansprüche, wobei jeder Parametervektor ($F^n(r)$) umfasst:

- mindestens einen optischen Parameter ($L(r)$), der für eine Lichtwegdifferenz entlang der Fortpflanzungsachse repräsentativ ist;

- einen optischen Parameter ($\alpha(r)$), der für die Absorbanz repräsentativ ist.

5. Verfahren nach einem der vorhergehenden Ansprüche, wobei der Gültigkeitsindikator $\left(\epsilon_{|F}^{n}\right)$ eine eventuell gewichtete Summe aus dem Fehlerkriterium $\left(\epsilon_{0|F}^{n}\right)$ und dem morphologischen Kriterium $\left(\epsilon_{10|F}^{n}\right)$ ist.

6. Verfahren nach Anspruch 5, wobei der Gültigkeitsindikator $\left(\epsilon_{|F}^{n}\right)$ eine gewichtete Summe aus dem Fehlerkriterium $\left(\epsilon_{0|F}^{n}\right)$ und dem morphologischen Kriterium $\left(\epsilon_{10|F}^{n}\right)$ ist, wobei die Gewichtung ($\gamma$) zwischen mindestens zwei aufeinanderfolgenden Iterationen variiert.

7. Verfahren nach einem der vorhergehenden Ansprüche, wobei:

- in Schritt b) ein Bild der Probe $\left(I_{0}^{\lambda}\right)$ in verschiedenen Spektralbändern ($\lambda$) erfasst wird;
- jeder Parametervektor mindestens einen optischen Parameter umfasst, der in einem der Spektralbänder definiert ist;

wobei das Verfahren derart beschaffen ist, dass:

- in Schritt iii) das komplexe Bild der modellierten Probe $\left(A_{10}^{\lambda,n}\right)$ in jedem Spektralband fortgepflanzt wird, so dass in jedem Spektralband ein Bild der modellierten Probe $\left(\hat{I}_{0}^{\lambda,n}\right)$ berechnet wird;
- Schritt iv) einen Vergleich des in Schritt iii) berechneten Bildes $\left(\hat{I}_{0}^{\lambda,n}\right)$ und des in Schritt b) erfassten Bildes $\left(I_{0}^{\lambda}\right)$ in jedem Spektralband ($\lambda$) umfasst;
- derart, dass der in Schritt iv) berechnete Gültigkeitsindikator auf der Grundlage der in Schritt iv) durchgeführten Vergleiche berechnet wird.

8. Verfahren nach Anspruch 7, wobei:

- bei den ersten Iterationen ein optischer Parameter so betrachtet wird, als variiere er nicht in Abhängigkeit vom Spektralband;
- der optische Parameter jenseits der ersten Iterationen als Variable in Abhängigkeit vom Spektralband betrachtet wird.

9. Verfahren nach einem der vorhergehenden Ansprüche, wobei in Schritt iii) die Berechnung des Bildes der modellierten Probe $\left(\hat{I}_{0}^{\lambda,n}\right)$ in der Detektionsebene ($P_0$) die Anwendung eines Faltungsprodukts durch einen Faltungskern ($K$) umfasst, wobei der Faltungskern eine räumliche Ausdehnung der Lichtquelle darstellt.

10. Verfahren nach einem der vorhergehenden Ansprüche, wobei in Schritt iii) die Berechnung des Bildes der modellierten Probe $\left(\hat{I}_{0}^{\lambda,n}\right)$ in der Detektionsebene ($P_0$) ein Integral eines elementaren Bildes umfasst, das in einem elementaren Spektralband definiert ist, wobei das Integral berechnet wird, um ein Emissionsspektralband der Lichtquelle zu berücksichtigen.

11. Vorrichtung zur Beobachtung einer Probe (10), umfassend:

- eine Lichtquelle (11), die dazu konfiguriert ist, eine einfallende Lichtwelle zu emittieren, um die Probe zu beleuchten;
- einen Probenträger (10s), der dazu konfiguriert ist, die Probe aufzunehmen;
- einen Bildsensor (16), der dazu konfiguriert ist, ein Bild der Probe zu erfassen, wenn die Probe auf dem Probenträger (10s) angeordnet ist;
- einen Prozessor (20), der dazu programmiert ist, Anweisungen auszuführen, die es ermöglichen, die Schritte i) bis vi) eines Verfahrens nach einem der vorhergehenden Ansprüche auf der Grundlage eines vom Bildsensor erfassten Bildes umzusetzen.

**Claims**

1. Method for observing a sample (10), the sample lying in a plane ($P_{10}$) of the sample defining radial coordinates (r), each radial coordinate corresponding to a pair of two coordinates (*x, y*) on two orthogonal axes (X, Y) of the plane of the sample, the method comprising the following steps:

   a) illuminating the sample using a light source (11), configured to emit an incident light wave (12) that propagates toward the sample along a propagation axis (Z);

   b) acquiring, using an image sensor (16), an image $\left(I_0^\lambda\right)$ of the sample (10), said image being formed in a detection plane ($P_0$), said image of the sample forming a hologram of the sample, the sample being placed between the light source (11) and the image sensor (16), such that the incident light wave experiences an optical path difference, parallel to the propagation axis (Z), on passing through the sample;
   c) processing the image acquired by the image sensor;

   the method being such that the processing of the acquired image comprises the following iterative steps:

   i) initializing a set ($\mathcal{F}^n$) of vectors ($F^n(r)$) of parameters, modelling the sample, the vectors of parameters being respectively defined at a plurality of radial coordinates (r), in the plane of the sample, each vector of parameters being associated with one radial coordinate (r), and comprising at least one component $\left(F_w^n(r)\right)$ representative of an optical parameter of the sample at said radial coordinate, at least one optical parameter being an optical path difference induced by the sample at said radial coordinate;

   ii) on the basis of the vectors ($F^n(r)$) of parameters, forming a complex image $\left(A_{10}^{\lambda,n}\right)$ of the modelled sample in the plane ($P_{10}$) of the sample;

   iii) applying a holographic propagation operator to the complex image of the modelled sample formed in the plane of the sample, in order to compute an image $\left(\hat{I}_0^{\lambda,n}\right)$ of the modelled sample in the detection plane;

   iv) comparing the image $\left(I_0^\lambda\right)$ acquired in step b) and the image $\left(\hat{I}_0^{\lambda,n}\right)$ computed in step iii), in order to compute a validity indicator $\left(\epsilon_{|\mathcal{F}}^n\right)$;

   v) updating all of the vectors of parameters, so as to make the validity indicator $\left(\epsilon_{|\mathcal{F}}^n\right)$ tend toward a preset value;
   vi) reiterating steps ii) to v) taking into account the vectors ($F^{n+1}(r)$) of parameters updated in step v);

   the method being **characterized in that**:

   step v) comprises computing a morphological criterion $\left(\epsilon_{10|\mathcal{F}}^n\right)$ from the vectors ($F^n(r)$) of parameters, the morphological criterion quantifying a deviation of the vectors of parameters, describing the sample, with respect to a morphological constraint of the sample, such that the validity indicator $\left(\epsilon_{|\mathcal{F}}^n\right)$ is obtained from a combination:

   - of an error criterion $\left(\epsilon_{0|\mathcal{F}}^n\right)$, determined from a comparison between the image acquired in step b) and the image obtained in step iii);

   - and of the morphological criterion $\left(\epsilon_{10|\mathcal{F}}^n\right)$.

2. Method according to Claim 1, wherein step v) comprises determining a gradient $\left(G_w^n(r)\right)$ of the validity indicator $\left(\epsilon_{|\mathcal{F}}^n\right)$ as a function of at least one parameter $\left(F_w^n(r)\right)$, such that the vectors ($F^{n+1}(r)$) of parameters are updated in order to decrease the validity indicator $\left(\epsilon_{|\mathcal{F}}^{n+1}\right)$ of the following iteration.

3. Method according to either one of Claims 1 and 2, wherein step v) implements an algorithm of gradient-descent type.

4. Method according to any one of the preceding claims, wherein each parameter vector ($F^n(r)$) comprises:

    - at least one optical parameter ($L(r)$) representative of an optical path difference along the propagation axis;
    - an optical parameter ($\alpha(r)$) representative of absorbance.

5. Method according to any one of the preceding claims, wherein the validity indicator $\left(\epsilon^n_{|\mathcal{F}}\right)$ is a sum, optionally weighted, of the error criterion $\left(\epsilon^n_{0|\mathcal{F}}\right)$ and of the morphological criterion $\left(\epsilon^n_{10|\mathcal{F}}\right)$.

6. Method according to Claim 5, wherein the validity indicator $\left(\epsilon^n_{|\mathcal{F}}\right)$ is a weighted sum of the error criterion $\left(\epsilon^n_{0|\mathcal{F}}\right)$ and of the morphological criterion $\left(\epsilon^n_{10|\mathcal{F}}\right)$, the weighting ($\gamma$) varying between at least two successive iterations.

7. Method according to any one of the preceding claims, wherein:

    - in step b), an image $\left(I^\lambda_0\right)$ of the sample is acquired in various spectral bands ($\lambda$);
    - each vector of parameters comprises at least one optical parameter defined in one of the spectral bands;

    the method being such that:

    - in step iii), the complex image $\left(A^{\lambda,n}_{10}\right)$ of the modelled sample is propagated in each spectral band, so as to compute, in each spectral band, one image $\left(\hat{I}^{\lambda,n}_0\right)$ of the modelled sample;
    - step iv) comprises a comparison, in each spectral band ($\lambda$), of the image $\left(\hat{I}^{\lambda,n}_0\right)$ computed in step iii) and of the image $\left(I^\lambda_0\right)$ acquired in step b);
    - such that the validity indicator computed in step iv) is computed on the basis of the comparisons made in iv).

8. Method according to Claim 7, wherein:

    - in first iterations, an optical parameter is considered to not vary as a function of the spectral band;
    - beyond first iterations, the optical parameter is considered to be variable as a function of the spectral band.

9. Method according to any one of the preceding claims, wherein, in step iii), the computation of the image $\left(\hat{I}^{\lambda,n}_0\right)$ of the modelled sample in the detection plane ($P_0$) comprises applying a convolution with a convolution kernel ($K$), the convolution kernel representing a spatial extent of the light source.

10. Method according to any one of the preceding claims, wherein, in step iii), the computation of the image $\left(\hat{I}^{\lambda,n}_0\right)$ of the modelled sample in the detection plane ($P_0$) comprises an integral of an elementary image, defined in an elementary spectral band, the integral being computed to take into account an emission spectral band of the light source.

11. Device for observing a sample (10), comprising:

    - a light source (11), configured to emit an incident light wave in order to illuminate the sample;
    - a sample holder (10s), configured to receive the sample;
    - an image sensor (16), configured to acquire an image of the sample when the sample is placed on the sample holder (10s);
    - a processor (20), programmed to execute instructions allowing steps i) to vi) of a method according to any one of the preceding claims to be implemented on the basis of an image acquired by the image sensor.

**Fig. 1A**

**Fig. 1B**

**Fig. 2**

**Fig. 3**

**Fig. 4A**

**Fig. 4B**

**Fig. 4C**

**Fig. 4D**

**Fig. 4E**

**Fig. 5**

## RÉFÉRENCES CITÉES DANS LA DESCRIPTION

**Documents brevets cités dans la description**

- WO 2008090330 A
- US 20120218379 A
- WO 2016189257 A
- WO 2017162985 A
- US 2017059468 A

**Littérature non-brevet citée dans la description**

- **THOMAS O.** Optimizing phase object reconstruction using an in-line digital holographic microscope and a reconstruction based on a Lorenz-Mie mode. SPIE, 24 Mai 2018, 10677